# EUROPEAN PATENT SPECIFICATION

(11) **EP 2 811 023 B1**
(45) Date of publication and mention of the grant of the patent: **24.10.2018**
(21) Application number: 13743799.2
(22) Date of filing: 01.02.2013
(51) Int. Cl.: C12N 15/12, C12N 15/861, C12N 7/01, A61K 35/12, C12N 15/63

(54) **VECTOR SIMULTANEOUSLY EXPRESSING DODECAMERIC TRAIL AND HSV-TK SUICIDE GENES, AND ANTICANCER STEM CELL THERAPEUTIC AGENT USING SAME**
VEKTOR ZUR GLEICHZEITIGEN EXPRESSION VON DODECAMERISCHEN PFAD- UND HSV-TK-SELBSTMORDGENEN SOWIE STAMMZELLENTHERAPEUTIKUM DAMIT
VECTEUR EXPRIMANT SIMULTANÉMENT TRAIL DODÉCAMÈRE ET DES GÈNES SUICIDES HSV-TK, ET AGENT THÉRAPEUTIQUE ANTICANCÉREUX À BASE DE CELLULES SOUCHES L'UTILISANT

(30) Priority: 01.02.2012 KR 20120010462
(43) Date of publication of application: 10.12.2014
(73) Proprietor: Postech Academy-Industry Foundation, Pohang-si, Gyeongsangbuk-do 790-784 (KR); Biod Co., Ltd., Daejeon 302-831 (KR)
(72) Inventor: SUNG, Young Chul, Pohang-si Gyeongsangbuk-do 790-758 (KR); KIM, Sae Won, Seoul 122-200 (KR); KIM, Su Jin, Gyeongju-si Gyeongsangbuk-do 780-922 (KR); PARK, Sang Hoon, Anyang-si Gyeonggi-do 431-083 (KR)
(74) Representative: Gassner, Birgitta
(86) International application number: PCT/KR2013/000849
(87) International publication number: WO 2013/115608

(56) References cited:
- WO-A1-2007/102690
- WO-A2-2009/028870
- KR-A- 20090 015 885
- CHRISTIAN BELTINGER ET AL: "TRAIL enhances thymidine kinase/ganciclovir gene therapy of neuroblastoma cells", CANCER GENE THERAPY, vol. 9, no. 4, 1 January 2002 (2002-01-01) , pages 372-381, XP055204571, DOI: 10.1038/sj.cgt.7700448
- S. W. KIM ET AL: "Complete Regression of Metastatic Renal Cell Carcinoma by Multiple Injections of Engineered Mesenchymal Stem Cells Expressing Dodecameric TRAIL and HSV-TK", CLINICAL CANCER RESEARCH, vol. 19, no. 2, 30 November 2012 (2012-11-30), pages 415-427, XP055079768, ISSN: 1078-0432, DOI: 10.1158/1078-0432.CCR-12-1568
- MOHR, ANDREA ET AL.: 'Mesenchymal stem cells expressing TRAIL lead to tumour growth inhibition in an experimental lung cancer model' JOURNAL OF CELLULAR AND MOLECULAR MEDICINE vol. 12, no. 6B, 29 March 2008, pages 2628 - 2643, XP002563288
- GRISEMDI, GIULIA ET AL.: 'Adipose-derived mesenchymal stem cells as stable source of tumor necrosis factor-related apoptosis-inducing ligand delivery for cancer therapy' CANCER RESEARCH vol. 70, no. 1, 13 April 2010, pages 3718 - 3729, XP055079759
- LOEBINGER, MICHAEL R. ET AL.: 'Mesenchymal stem cell delivery of TRAIL can eliminate metastatic cancer' CANCER RESEARCH vol. 69, no. 10, 12 May 2009, pages 4134 - 4142, XP055079762
- LOEBINGER, MR. ET AL.: 'TRAIL-expressing mesenchymal stem cells kill the putative cancer stem cell population' BRITISH JOURNAL OF CANCER vol. 103, no. 11, 09 November 2010, pages 1692 - 1697, XP055079764
- UCHBORI, RYOSUKE ET AL.: 'Retroviral vector-producing mesenchymal stem cells for targeted suicide cancer gene therapy' THE JOURNAL OF GENE MEDICINE vol. 11, no. 5, 09 March 2009, pages 373 - 381, XP055079765
- MATUSKOVA, MIROSLAVA ET AL.: 'HSV-tk expressing mesenchymal stem cells exert bystander effect on human glioblastoma cells' CANCER LETTERS vol. 290, no. 1, 01 April 2010, pages 58 - 67, XP026929875
- KIM, SAE WON ET AL.: 'Complete regression of metastatic renal cell carcinoma by multiple injections of engineered mesenchymal stem cells expressing dodecameric TRAIL and HSV-TK' CLINICAL CANCER RESEARCH vol. 19, no. 2, 30 November 2012, pages 415 - 427, XP055079768

<110> POSTECH ACADEMY-INDUSTRY FOUNDATION Biod Co.,LTD.
<120> Vector coexpressing dodecameric TRAIL and suicide gene HSV-TK, and anticancer stem cell medicine using thereof
<130> OPA13015PCT
<150> 10-2012-0010462
   <151> 2012-02-01
<160> 6
<170> KopatentIn 2.0
<210> 1
   <211> 1341
   <212> DNA
   <213> Artificial Sequence
<220>
   <223> sequence for codon optimized dodecameric TRAIL
<400> 1
   atggacgcca tgaagcgcgg cctgtgctgc gtgctgctgc tgtgcggcgc cgtgtttgtg      60
   agccccagcg ctagcggcgg aggaaattcc gcagagatga agacttacag ccaccggact     120
   actccttccg cctgcacact ggtcatgtgt tcatccgtcg agtctgggct ccccggccgg     180
   gacgggaggg acggacgcga ggggccaagg ggcgagaagg gcgaccctgg actccccggg     240
   gccgccggac aggcaggaat gcccggccaa gctggacctg tggggcccaa aggagacaac     300
   gggtccgtgg gggagccagg accaaaggga gacactggcc ctagcggccc ccctgggcca     360
   cctggtgtgc ctggaccagc tggtagggag ggccccctgg gcaaacaggg aaacatcggg     420
   ccccagggga agcccggacc aaagggagag gccggaccta agggtgaagt cggggcccca     480
   ggcatgcagg gctccgctgg cgcccgcggg cttgcagggc cgaagggcga acgtggtgtg     540
   cctggggagc ggggggtccc tggtaacgct ggcgctgctg gatccgccgg agctatgggg     600
   ccacaaggtt ctcctggggc caggggcccg ccaggcttga agggcgacaa aggcataccc     660
   ggtgacaagg gagccaaagg cgagagcggg cttcctgacg tggccagcct gcgccaacag     720
   gtggaagcac tccagggaca ggttcaacat cttcaggccg ccttctccca gtacaaaaag     780
   gttgagcttt ttcctaacgg gggcggcggc aattcagggc gcgccgcggc cgctgtgagg     840
   gaaaggggac cacagcgcgt ggcagcccac atcacaggca caagagggag aagcaatacc     900
   ctgagcagcc caaattcaaa gaatgagaag gcactgggga ggaaaatcaa ctcatgggaa     960
   agttcacggt ctggacatag tttcctgtct aacctgcacc tgagaaacgg cgagctggtc    1020
   attcacgaga aggggtttta ctacatctat agtcagacat acttcagatt tcaggaagag    1080
   attaaggaga acacaaaaaa tgacaagcag atggtgcagt atatctacaa atataccagc    1140
   tacccagacc ctatcctgct catgaagtct gcccggaaca gctgttggtc caaggacgct    1200
   gaatacggac tctacagcat ctaccagggg ggaatcttcg agctgaaaga aaatgaccgg    1260
   attttcgtga gcgtgaccaa cgagcacctg attgatatgg accacgaggc cagcttcttc    1320
   ggggcattcc tggtgggttg a                                              1341
<210> 2
   <211> 1131
   <212> DNA
   <213> Artificial Sequence
<220>
   <223> sequence for codon optimized HSV-TK
<400> 2
   atggccagct acccctgtca ccagcacgcc agcgccttcg accaggccgc tagaagcaga      60
   ggccacagca acagaagaac cgccctgaga cccagaagac agcaggaggc cacagaggtg     120
   agactggagc agaagatgcc caccctgctg agagtgtaca tcgatggacc ccacggcatg     180
   ggcaagacca caacaaccca gctgctggtg gccctgggca gcagagacga catcgtgtac     240
   gtgcccgagc ccatgaccta ctggcaggtg ctgggagcca gcgagaccat cgccaacatc     300
   tacaccacac agcacagact ggaccagggc gagatcagcg ccggcgacgc tgccgtggtg     360
   atgaccagcg cccagatcac aatgggcatg ccctacgccg tgaccgatgc cgtgctggct     420
   ccccacgtgg gcggagaggc cggcagcagc cacgcccctc cccctgccct gaccctgatc     480
   ttcgacagac accccatcgc cgccctgctg tgctaccccg ccgctagata cctgatgggc     540
   agcatgacac cccaggccgt gctggccttc gtggccctga tcccccctac cctgcccggc     600
   accaacatcg tgctgggcgc cctgcccgag gacagacaca tcgacagact ggctaagaga     660
   cagagacccg gcgagagact ggacctggcc atgctggccg ccatcagaag agtgtacggc     720
   ctgctggcca acaccgtgag atacctgcag ggaggcggca gctggtggga ggactggggc     780
   cagctgagcg gcaccgccgt gcctccccag ggcgccgagc cccagagcaa cgccggccct     840
   agaccccaca tcggcgacac cctgttcacc ctgtttagag cccccgagct gctggccccc     900
   aacggcgacc tgtacaacgt gttcgcctgg gccctggacg tgctggccaa gagactgaga     960
   cccatgcacg tgttcatcct ggactacgac cagagccccg ccggctgcag agatgccctg    1020
   ctgcagctga ccagcggcat ggtgcagacc cacgtgacca cacccggcag catccccacc    1080
   atctgcgacc tggccagaac ctttgccaga gagatgggcg aggccaactg a             1131
<210> 3
   <211> 586
   <212> DNA
   <213> Artificial Sequence
<220>
   <223> sequence for IRES
<400> 3
   gaattccccc tctccctccc ccccccctaa cgttactggc cgaagccgct tggaataagg 60
   ccggtgtgcg tttgtctata tgttattttc caccatattg ccgtcttttg gcaatgtgag 120
   ggcccggaaa cctggccctg tcttcttgac gagcattcct aggggtcttt cccctctcgc 180
   caaaggaatg caaggtctgt tgaatgtcgt gaaggaagca gttcctctgg aagcttcttg 240
   aagacaaaca acgtctgtag cgaccctttg caggcagcgg aaccccccac ctggcgacag 300
   gtgcctctgc ggccaaaagc cacgtgtata agatacacct gcaaaggcgg cacaacccca 360
   gtgccacgtt gtgagttgga tagttgtgga aagagtcaaa tggctctcct caagcgtatt 420
   caacaagggg ctgaaggatg cccagaaggt accccattgt atgggatctg atctggggcc 480
   tcggtgcaca tgctttacgt gtgtttagtc gaggttaaaa aacgtctagg ccccccgaac 540
   cacggggacg tggttttcct ttgaaaaaca cgatgataat atggcg 586
<210> 4
   <211> 75
   <212> DNA
   <213> Artificial Sequence
<220>
   <223> sequence for codon optimized tissue plasminogen activator
<400> 4
   atggacgcca tgaagcgcgg cctgtgctgc gtgctgctgc tgtgcggcgc cgtgtttgtg 60
   agccccagcg ctagc 75
<210> 5
   <211> 750
   <212> DNA
   <213> Artificial Sequence
<220>
   <223> sequence for codon optimized surfactant protein D
<400> 5
   ggcggaggaa attccgcaga gatgaagact tacagccacc ggactactcc ttccgcctgc 60
   acactggtca tgtgttcatc cgtcgagtct gggctccccg gccgggacgg gagggacgga 120
   cgcgaggggc caaggggcga gaagggcgac cctggactcc ccggggccgc cggacaggca 180
   ggaatgcccg gccaagctgg acctgtgggg cccaaaggag acaacgggtc cgtgggggag 240
   ccaggaccaa agggagacac tggccctagc ggcccccctg ggccacctgg tgtgcctgga 300
   ccagctggta gggagggccc cctgggcaaa cagggaaaca tcgggcccca ggggaagccc 360
   ggaccaaagg gagaggccgg acctaagggt gaagtcgggg ccccaggcat gcagggctcc 420
   gctggcgccc gcgggcttgc agggccgaag ggcgaacgtg gtgtgcctgg ggagcggggg 480
   gtccctggta acgctggcgc tgctggatcc gccggagcta tggggccaca aggttctcct 540
   ggggccaggg gcccgccagg cttgaagggc gacaaaggca tacccggtga caagggagcc 600
   aaaggcgaga gcgggcttcc tgacgtggcc agcctgcgcc aacaggtgga agcactccag 660
   ggacaggttc aacatcttca ggccgccttc tcccagtaca aaaaggttga gctttttcct 720
   aacgggggcg gcggcaattc agggcgcgcc 750
<210> 6
   <211> 516
   <212> DNA
   <213> Artificial Sequence
<220>
   <223> sequence for codon optimized TRAIL
<400> 6
   gcggccgctg tgagggaaag gggaccacag cgcgtggcag cccacatcac aggcacaaga 60
   gggagaagca ataccctgag cagcccaaat tcaaagaatg agaaggcact ggggaggaaa 120
   atcaactcat gggaaagttc acggtctgga catagtttcc tgtctaacct gcacctgaga 180
   aacggcgagc tggtcattca cgagaagggg ttttactaca tctatagtca gacatacttc 240
   agatttcagg aagagattaa ggagaacaca aaaaatgaca agcagatggt gcagtatatc 300
   tacaaatata ccagctaccc agaccctatc ctgctcatga agtctgcccg gaacagctgt 360
   tggtccaagg acgctgaata cggactctac agcatctacc aggggggaat cttcgagctg 420
   aaagaaaatg accggatttt cgtgagcgtg accaacgagc acctgattga tatggaccac 480
   gaggccagct tcttcggggc attcctggtg ggttga 516

## Description

### [Technical Field]

The present invention relates to a vector coexpressing dodecameric TNF related apoptosis inducing ligand (TRAIL) and Herpes simplex virus thymidine kinase (HSV-TK) suicide genes, and an anticancer stem cell therapeutic agent using the same. Particularly, the present invention relates to a DNA cassette comprising a nucleotide sequences encoding dodecameric TRAIL and a suicide gene nucleotide sequence, a recombinant expression vector comprising the DNA cassette, a recombinant adenovirus prepared by using the recombinant expression vector, a host cell transduced with the recombinant adenovirus, a composition for treating cancer comprising the host cell, and a method for treating cancer comprising the step of administering the composition for treating cancer to a subject.

### [Background Art]

Mesenchymal stem cells (MSCs) are pluripotent adult stem cells that can differentiate into osteoblasts, chondrocytes, and adipocytes, and are widely used as a therapeutic agent for many different types of diseases for the purpose of tissue regeneration. In addition, MSCs are known to have the property of specifically tracking and migrating toward tumor sites in vivo, and recently, have emerged as targeted-delivery vehicles for anticancer agents (Aboody et al., Proc Natl Acad Sci, 97: 12846, 2000). Many studies have been conducted on targeted therapy of metastatic cancer using the tumor-tropic nature of MSCs. Practically, when mice bearing metastatic cancer received systemic administration of MSCs expressing anticancer cytokine IL-12 or IFN-beta, or oncolytic virus, the size of metastatic lesion was decreased and survival of the mice was extended (Shah et al. Advanced Drug Delivery Reviews, 2011).

TRAIL is a TNF (tumor necrosis factor) family protein, and is known to induce selective apoptosis of tumor cells (Wiley et al., Immunity, 3(6):673-682, 1995). In fact, TRAIL was found to exert cytotoxicity on various cancer cell lines without damaging healthy tissues (Ashkenazi et al., J. Clin. Invest., 104:155-162, 1999; Walczak et al., Nat. Med., 5:157-163, 1999), owing to high-level expression of decoy receptors 1 and 2 (DcR1 and DcR2) by normal cells on their cell surfaces (Sheridan et al., Science, 277:81 8-821, 1997). DcR1 and DcR2 bind to TRAIL, but do not transduce TRAIL-mediated apoptotic signals due to their absence of intracellular signaling domains. Based on its ability to induce tumor cell-specific apoptosis, TRAIL has emerged as a promising therapeutic agent against cancer. In the prior art, the present inventors developed a dodecameric TRAIL by linking a secretion signal sequence of tPA (tissue plasminogen activation) and a dodecamer-forming domain of SPD (surfactant protein D) to the amino terminus of the extracellular domain (amino acid 114-281) of TRAIL, that can bind to the TRAIL receptors DR4 and DR5, for achieving effective anticancer gene therapy utilizing TRAIL (International Publication No. WO2007/102690).

Suicide gene therapy is one of the anticancer gene therapies, and is a method of delivering suicide genes such as HSV-TK (Herpes simplex virus thymidine kinase), cytosine deaminase, nitroreductase, carboxylesterase, cytochrome P450, or PNP (Purine nucleoside phosphorylase) directly to tumor cells. The suicide gene expresses one of the above-mentioned enzymes, and this enzyme converts an injected non-toxic prodrug into a cytotoxic substance through enzymatic reaction. The cytotoxic substance is transferred from the cells expressing the suicide gene to adjacent cells via gap junctions to induce apoptosis of the neighboring cells, and this phenomenon is known as the bystander effect. Importantly, when a prodrug is systemically administered to the living body after inducing the expression of the suicide gene in a tumor tissue, the prodrug is converted into a cytotoxic substance only in the vicinity of tumor cells and subsequently induces their apoptosis, without eliciting significant damages to normal tissues (The FASEB journal, 23:1584, 2009).

Among various types of suicide genes, HSV-TK is the most widely used suicide gene, and its efficacy and safety have already been demonstrated in phase III clinical trials. HSV-TK phosphorylates a prodrug ganciclovir (GCV), and the phosphorylated ganciclovir triphosphate (GCV-3P) incorporates into DNA to prevent normal DNA synthesis, leading to cell apoptosis (Moolten et al., Cancer Res. 46: 5276, 1986). The action of GCV-3P is specific to rapidly proliferating cells, and thus this method can be used to selectively eliminate tumor cells while sparing normal cells. In practice, when HSV-TK is transferred to a local tumor site using adenovirus or lentivirus, and then GCV is administered, tumor-specific apoptosis can be expected. To further improve the tumor specificity, tumor-specific promoters or tropism-modifying strategies of viral vectors are used.

TRAIL and HSV-TK are considered as safe candidates for targeted cancer therapy in terms of their ability to induce tumor-specific apoptosis, and they have been applied for the treatment of metastatic cancers utilizing engineered MSCs. However, anticancer efficacy of the TRAIL or HSV-TK therapy alone has been considered moderate, leading to the limited clinical success in the treatment of cancer (Loebinger et al., Cancer Res, 69: 4134, 2009; Miletic et al. Molecular Ther, 15: 1373, 2007).

### [DISCLOSURE]

### [Technical Problems]

To develop highly effective targeted anticancer gene therapy, the present inventors constructed a vector coexpressing dodecameric TRAIL and HSV-TK suicide genes and subsequently produced a recombinant adenovirus coexpressing these genes. When the recombinant adenovirus was delivered to stem cells, transduced cells exhibited superior apoptosis-inducing ability against various tumor cells. MSCs coexpressing dodecameric TRAIL and HSV-TK showed combinatory anticancer effects, compared to those expressing TRAIL, dodecameric TRAIL, or HSV-TK alone. Furthermore, when MSCs coexpressing dodecameric TRAIL and HSV-TK were systemically administered to mice bearing pulmonary metastasis, MSCs were found to be specifically localized in the vicinity of tumor nodules, and the size as well as the number of metastatic nodules was markedly reduced, leading to significant improvement of mice survival. More importantly, repeated administrations of MSCs coexpressing dodecameric TRAIL and HSV-TK resulted in further enhancement of anticancer effects, and after three injections, all metastatic tumor nodules were completely eliminated in mice, thereby completing the present invention.

### [Technical Solution]

An object of the present invention is to provide a stem cell coexpressing dodecameric TNF related apoptosis inducing ligand (TRAIL) and a suicide gene nucleotide sequence.

Another object of the present invention is to provide a stem cell as host cell which is transduced with the recombinant adenovirus.

Still another object of the present invention is to provide a composition for treating cancer comprising the host cell.

Still another object of the present invention is to provide a method for treating cancer comprising the step of administering the composition for treating cancer to a subject suspected of having cancer.

### [Advantageous Effect]

The stem cell therapy coexpressing dodecameric TRAIL and HSV-TK by introduction of the DNA cassette of the present invention has more potent anticancer effects than the known therapy, and thus can be effectively used in the treatment of many different types of solid tumors and metastatic tumors.

### [Description of Figures]

FIG. 1 shows a schematic illustration of a DNA cassette (dTRAIL-TK) composed of human dodecameric TRAIL sequence, IRES sequence, and HSV-TK sequence;
FIG. 2 shows the expression of TRAIL and HSV-TK by dTRAIL-TK DNA cassette-introduced MSCs (MSC/dTRAIL-TK);
FIG. 3 shows the cell survival and TRAIL expression by dTRAIL-TK DNA cassette-introduced MSCs (MSC/dTRAIL-TK) upon the treatment of GCV;
FIG. 4 shows the cytotoxic effects of mesenchymal stem cells coexpressing dodecameric TRAIL and HSV-TK (MSC/dTRAIL-TK) against RENCA cells *in vitro;*
FIG. 5 shows the number of pulmonary metastatic nodules in a murine metastatic renal cell carcinoma model upon MSC/dTRAIL-TK treatment;
FIG. 6 shows the survival rate of mice with metastatic renal cell carcinoma following the administration of MSC/dTRAIL-TK; and
FIG. 7 shows the survival rate of mice with metastatic renal cell carcinoma following the repeated administrations of MSC/dTRAIL-TK.

### [Best Mode]

In one aspect to achieve the above objects, the present invention provides a DNA cassette comprising a nucleotide sequence encoding a dodecameric TNF related apoptosis inducing ligand (TRAIL) and a suicide gene nucleotide sequence.

As used herein, the term "dodecameric TNF related apoptosis inducing ligand (TRAIL)" is intended to refer to a secretory TRAIL protein having a dodecameric structure by multimerization of expressed TRAIL proteins, and it may be a dodecameric TRAIL which is prepared by a method described in International Publication No. WO2007/102690 and Korean Patent Publication No. 10-2009-0015885. The dodecameric TRAIL protein of the present invention was found to show higher apoptosis-inducing activity against tumor cells compared to monomeric or trimeric TRAIL. In the present invention, a nucleotide sequence encoding a dodecamer-forming domain was located at 5' upstream of the nucleotide sequence encoding the TRAIL protein to produce TRAIL as a dodecamer, and a nucleotide sequence encoding a secretion signal sequence was located at 5' upstream of the dodecamer-forming domain for extracellular secretion of the protein.

Although the dodecameric TRAIL was used in the present invention, a trimeric or multimeric TRAIL can also be included in the scope of the present invention, based on the combinatory anticancer effects exerted by the coexpression of dTRAIL and HSV-TK. The nucleotide sequence encoding dodecameric TRAIL of the present invention may include a nucleotide sequence encoding a secretion signal sequence, a nucleotide sequence encoding a dodecamer-forming domain, and a nucleotide sequence encoding TRAIL.

As used herein, the term "TNF related apoptosis inducing ligand (TRAIL)" is one of the proteins that function as a ligand to induce the process of cell death, called apoptosis. In the present invention, the nucleotide sequence encoding TRAIL may be a gene encoding TRAIL protein derived from human, monkey, rat, mouse, or other species, and human TRAIL gene may be preferably used.

Since the protein expressed from the nucleotide sequence encoding TRAIL of the present invention has a reactivity with the TRAIL receptor DR4 or DR5, only a part of the nucleotide sequence encoding TRAIL can be used. Preferably, a nucleotide sequence encoding 114 to 281 amino acids of TRAIL may be used. The nucleotide sequence encoding TRAIL can be easily prepared by those illustrated in the previous articles using the information available in GenBank (GeneID: 8743, Pitti. R. M. et al., J. Biol. Chem. 271: 12687, 1996).

In the present invention, the secretion signal sequence serves to secrete the TRAIL protein to outside the cells. Since TRAIL is expressed on the cell surface, TRAIL lacks its own secretion signal sequence. The extracellular domain of TRAIL is known to induce apoptotic signaling by TRAIL protein. In the present invention, the secretion signal sequence is used for extracellular secretion of the dodecameric TRAIL protein, thereby increasing the anticancer effects thereof. The nucleotide sequence encoding the secretion signal sequence is located 5' upstream of the nucleotide sequence encoding the dodecamer-forming domain. Examples of the secretion signal sequence may include, but are not limited to, tPA, HSV, gDs, SEC2, SEC(CV) or the like with preference for tPA (tissue plasminogen activator).

In one embodiment of the present invention, tPA was used as the secretion signal sequence. tPA is a secretion signal sequence having excellent capability of inducing secretion and is able to secrete the dodecameric TRAIL protein of the present invention outside cells more effectively.

In the present invention, the dodecamer-forming domain assists the dodecamerization of TRAIL proteins for the preparation of a stable form of the TRAIL protein through multimerization thereof. In order to achieve this, the nucleotide sequence encoding the dodecamer-forming domain was allowed to locate 5' upstream of the nucleotide sequence encoding the TRAIL protein. The dodecamer-forming domain is, but not limited to, preferably SPD (surfactant protein D).

As described above, the nucleotide sequence encoding the dodecameric TRAIL of the present invention may be composed of the nucleotide sequence encoding the secretion signal sequence, the nucleotide sequence encoding the dodecamer-forming domain, and the nucleotide sequence encoding TRAIL. In one embodiment of the present invention, the nucleotide sequence encoding tPA secretion signal sequence of SEQ ID NO. 4, the nucleotide sequence encoding SPD dodecamer-forming domain of SEQ ID NO. 5, and the nucleotide sequence encoding TRAIL of SEQ ID NO. 6 were chemically synthesized to prepare a codon-optimized nucleotide sequence encoding human dodecameric TRAIL, and finally, a tPA-SPD-TRAIL DNA cassette of SEQ ID NO. 1 was prepared. Preferably, the nucleotide sequence encoding the dodecameric TRAIL of the present invention may have the nucleotide sequence of SEQ ID NO. 1.

As used herein, the term "suicide gene" is a gene capable of inducing cell apoptosis, and it may be, but is not limited to, preferably HSV-TK (Herpes simplex virus thymidine kinase), cytosine deaminase, nitroreductase, carboxylesterase, cytochrome P450 or PNP (Purine nucleoside phosphorylase), and more preferably, HSV-TK. In addition, the HSV-TK may be preferably a codon-optimized HSV-TK represented by SEQ ID NO. 2.

As the nucleotide sequences encoding dodecameric TRAIL and HSV-TK protein of the present invention, the sequences identical to those of the wild-type may be used as they is, but codon-optimized nucleotide sequences of dodecameric TRAIL and HSV-TK protein may be preferably used so that protein expression frequency is increased in mammalian cells, especially, in human cells. The term "codon-optimized sequence" or "codon optimization", as used herein, is intended to refer to the substitution of some of the amino acid codons encoding a protein of interest (e.g., the dodecameric TRAIL or HSV-TK protein of the present invention) with such codons as increase the expression level of the protein of interest in mammalian cells, especially in human cells. Various combinations of some of the amino acid codons to be substituted can be applied by those skilled in the art. In the present invention, one or more of the codons encoding the amino acids of dodecameric TRAIL and HSV-TK protein, in detail, for alanine (Ala: A), arginine (Arg: R), asparagine (Asn: N), aspartate (Asp: D), cysteine (Cys: C), glutamine (Gln: Q), glutamate (Glu: E), glycine (Gly: G), histidine (His: H), isoleucine (Ile: I), leucine (Leu: L), lysine (Lys: K), phenylalanine (Phe: F), proline (Pro: P), serine (Ser: S), threonine (Thr: T), valine (Val: V) and tyrosine (Tyr: Y) are substituted with those recognized as a higher frequency in human cells.

In a specific embodiment of the present invention, 1 to 23 nucleotides of the nucleotide sequence encoding tPA secretion signal sequence which constitutes the nucleotide sequence encoding dodecameric TRAIL were subjected to codon optimization, and the resulting sequence is represented by SEQ ID NO. 4, 78 to 314 nucleotides of the nucleotide sequence encoding the dodecamer-forming domain SPD were subjected to codon optimization, and the resulting sequence is represented by SEQ ID NO. 5, and the codon-optimized nucleotide sequence encoding TRAIL is represented by SEQ ID NO. 6. Therefore, the codon-optimized nucleotide sequences encoding dodecameric TRAIL and HSV-TK are represented by SEQ ID NOs. 1 and 2, respectively. Accordingly, the codon-optimized nucleotide sequences encoding dodecameric TRAIL and HSV-TK resulted in higher protein expression levels compared to those induced by wild-type sequences, and thus have excellent capability of inducing secretion of anticancer genes.

The DNA cassette of the present invention may further include a transcription/translation initiation sequence between the nucleotide sequence encoding the dodecameric TRAIL and the suicide gene nucleotide sequence. This is a sequence for coexpressing two genes in one vector, an IRES sequence, a 9-nt sequence or a dual promoter system can be preferably used. More preferably, IRES sequence can be used.

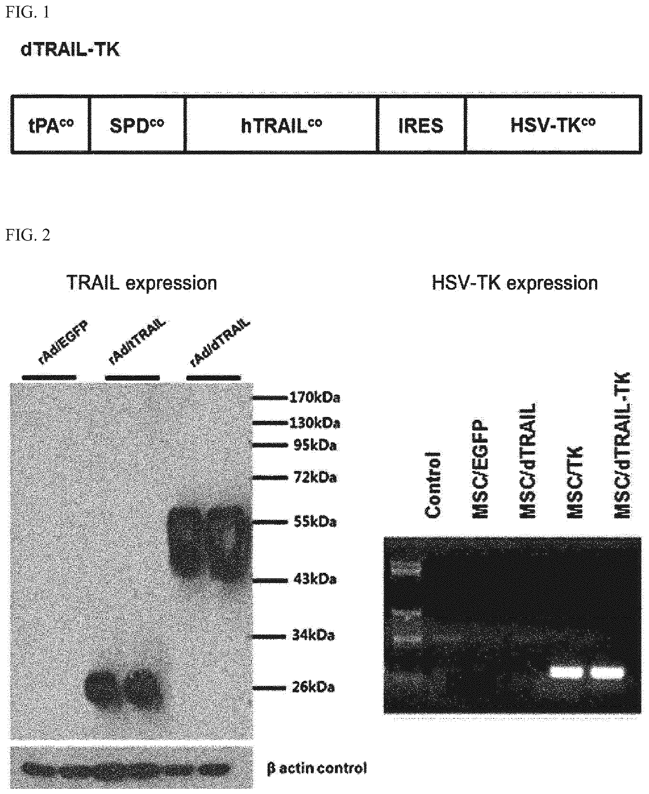

As used herein, the term "IRES", also called internal ribosome entry site, means a specific region found in mRNA, to which ribosome directly binds so as to allow multiple proteins to be synthesized from a single mRNA in eukaryotic cell. IRES functions to coexpress the dodecameric TRAIL and the suicide gene of the present invention. In one embodiment of the present invention, IRES sequence represented by SEQ ID NO. 3 and the nucleotide sequences encoding dodecameric TRAIL and HSV-TK were chemically synthesized to prepare a dTRAIL-IRES-TK cassette (FIG 1).

Described herein is a recombinant expression vector comprising the DNA cassette.

The vector, which describes a recombinant expression vector capable of expressing a protein of interest, that is, the dodecameric TRAIL and suicide gene proteins, in a host cell, may include essential regulatory elements to which a gene insert (e.g., the DNA cassette) is operably linked in such a manner as to be expressed.

As used herein, the term "operably linked" means that there is a functional linkage between a nucleotide expression control sequence and a nucleotide sequence encoding a target protein, in such a manner as to perform general functions. The operable linkage to the recombinant expression vector may be prepared using a genetic recombinant technique that is well known in the art, and site-specific DNA cleavage and ligation may be carried out using enzymes that are generally known in the art. A vector may include expression regulatory elements, such as a promoter, an operator, an initiation codon, a stop codon, a polyadenylation signal and an enhancer. Both the initiation codon and the stop codon are generally regarded as a part of the nucleotide sequence encoding the target protein and are necessary in order to be functional in an individual to whom a genetic construct has been administered, and must be in frame with the coding sequence.

The vector of the present invention may include plasmid vectors, cosmid vectors, bacteriophage vectors, and viral vectors, but are not limited thereto, with preference for viral vectors. Of them, adenoviral vectors are more preferable. An adenoviral vector, which is one of the most prevalent vectors for use in gene therapy of cancer patients, shows excellent efficiency in transfection into various cells.

The recombinant adenovirus used in the present invention is type 5 and is incapable of self-replication due to the deficiency of the E1A gene essential for replication. In one embodiment of the present invention, the DNA cassette prepared by chemically synthesizing the nucleotide sequences encoding dodecameric TRAIL, IRES sequence, and HSV-TK sequence were inserted into an adenovirus shuttle vector so as to prepare a recombinant expression vector capable of coexpressing dodecameric TRAIL and HSV-TK.

Described herein is a recombinant adenovirus which is prepared by using recombinant expression vector.

The recombinant adenovirus can be prepared by introducing the adenovirus-derived recombinant expression vector including the DNA cassette of the present invention into a packaging cell line, culturing the packaging cells, and harvesting the recombinant adenovirus produced from the packaging cells.

The recombinant adenovirus includes an inducible adenovirus which includes an inducible promoter to control expression in response to a particular compound. The inducible promoter is CMV-Tet10 and the expression is induced by tetracycline. A tetracycline-inducible system is one of the most widely used inducible systems in vivo and is economically advantageous with the production of no significant side effects.

In still another aspect, the present invention provides a host cell which is transduced with the recombinant adenovirus. The host cell of the present invention coexpresses dodecameric TRAIL and HSV-TK so as to kill tumor cells and to have excellent anticancer effects, and thus it can be widely used in cell therapy for cancer treatment.

As the host cell, a host cell widely known in the art can be used. Any host cell usable in cell therapy can be used, and stem cells are preferred. If the host cell is a stem cell, it can be used in stem cell therapy for cancer treatment.

As used herein, the term "stem cell" means a cell having a potential for both self-renewal and differentiation into many different types of cells. In the present invention, the stem cell is preferably an embryonic stem cell or an adult stem cell, and more preferably, an adult stem cell, MSC, and most preferably, a human MSC having tumor-tropic property.

Owing to their tumor-tropic nature MSC, MSCs coexpressing dodecameric TRAIL and HSV-TK can preferentially migrate toward tumors, and stably secrete dodecameric TRAIL, and HSV-TK converts a prodrug into a cytotoxic substance, which is transferred to adjacent tumor cells. In particular, dodecameric TRAIL is more stable as a secretory form and has higher capacity to induce the apoptosis of tumor cells compared to monomeric TRAIL, and thus it is more useful in the preparation of cell therapy product as well as direct injection thereof into tumor cells. In addition, MSCs coexpressing dodecameric TRAIL and HSV-TK were found to show combinatory anticancer effects, compared to those expressing dodecameric TRAIL or HSV-TK alone.

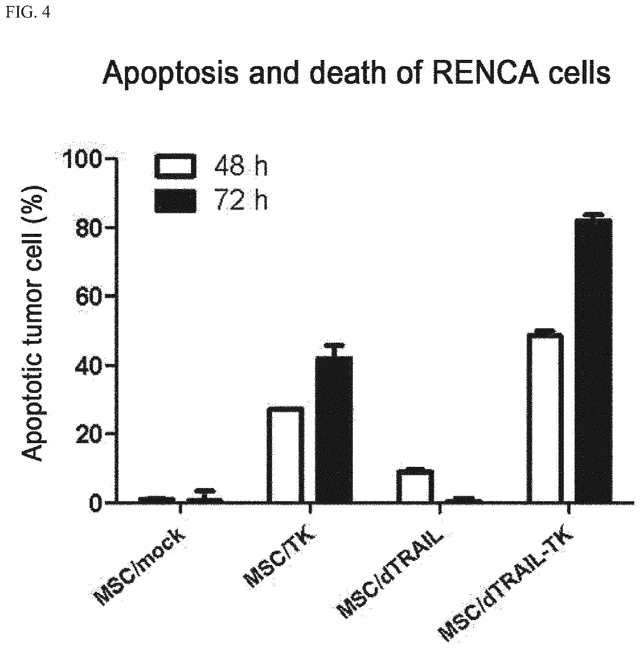

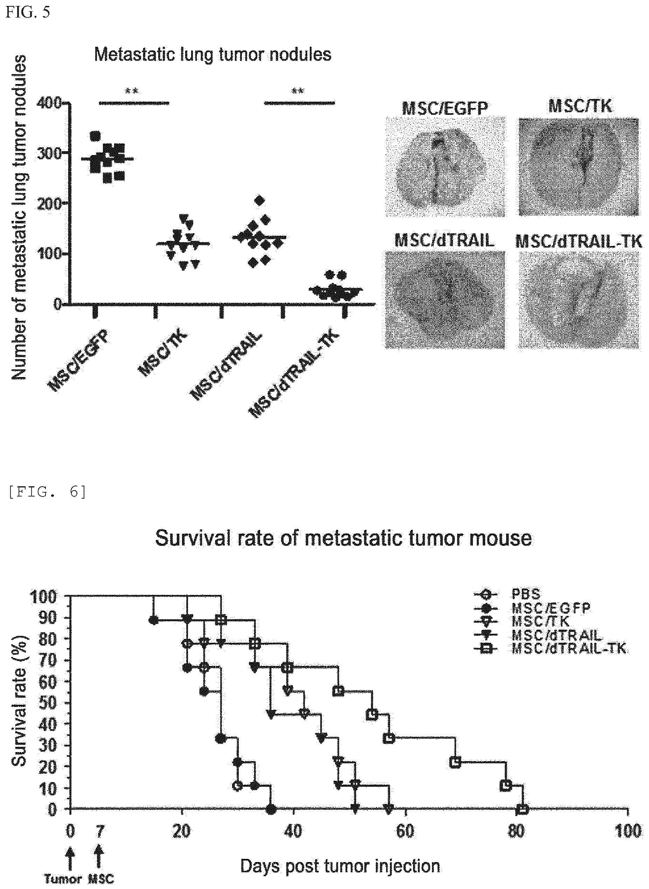

In one embodiment of the present invention, MSCs coexpressing dodecameric TRAIL and HSV-TK were prepared using the recombinant adenovirus. It was found that when MSCs were cocultured with tumor cells in vitro, the apoptosis of tumor cells was remarkably increased (FIG. 4), and the administration of engineered MSCs significantly reduced the number of metastatic tumor nodules (FIG. 5) and prolonged the survival of mice bearing metastatic renal cell carcinoma mouse (FIG. 6), suggesting that the cells of the present invention, in particular, stem cells can be used in the anticancer cell therapy for the treatment of cancer.

In still another aspect, the present invention provides a composition for treating cancer including the host cell. As described above, the host cell coexpresses the dodecameric TRAIL and the suicide gene protein, which act in a combinatorial manner to increase anticancer effects. Therefore, the host cell has anticancer therapeutic effects.

In addition to the host cell, the recombinant expression vector capable of coexpressing dodecameric TRAIL and HSV-TK protein of the present invention and the recombinant adenovirus prepared by using the vector can be also included as an active ingredient in the therapeutic composition for cancer of the present invention.

Cancer which can be treated by the composition of the present invention includes all types of common cancer diseases, and cancers of which apoptosis is induced by the cell therapeutic agent of the present invention are included without limitation. Examples thereof may include all types of cancers generally known, such as liver cancer, stomach cancer, colon cancer, breast cancer, lung cancer, pancreatic cancer, colorectal cancer, renal cancer, breast cancer, cervical cancer, prostate cancer, ovarian cancer, thyroid cancer, and include solid tumors and metastatic tumors. Metastatic tumors may be preferred. In one embodiment of the present invention, a metastatic carcinoma mouse model by renal carcinoma cell RENCA was used to examine cancer therapeutic effects (FIGs. 5 to 7).

The composition of the present invention includes a pharmaceutically acceptable carrier, and can be prepared into various dosage forms including tablets, troches, capsules, elixirs, suspensions, syrups, wafers, injections, etc. As long as it is pharmaceutically acceptable, any form of solvents, dispersing media, coating agents, antibacterial or antifungal agents, isotonics, and absorption retardants may be used as a carrier. These media and materials for effective use in pharmaceutically active ingredients are well known in the art.

Depending on the purpose thereof, the composition can be formulated into a suitable pharmaceutical composition.

In a specific embodiment of the present invention, the vector expressing dodecameric TRAIL and HSV-TK protein of the present invention, or an adenovirus or a stem cell prepared by using the vector is provided as a therapeutic composition for cancer. With this therapeutic composition, mammals including humans can be injected.

In a specific embodiment, the composition including dodecameric TRAIL and HSV-TK protein of the present invention may be administered via oral routes or non-oral routes, such as intramuscular, intravenous, intraarterial, peritoneal, subcutaneous, and intradermal routes. Preferable administration mode and formulation are an intravenous injection, a subcutaneous injection, an intradermal injection, an intramuscular injection, instillation, and an intratumoral injection. The composition may be administered in a single dose or in multiple doses. In particular, the composition of the present invention may be administered together with GCV (ganciclovir) or after administration of GCV.

Also, the composition must be administered in a pharmaceutically effective amount. The pharmaceutically effective amount is determined depending on the mode and frequency of administration, the kind and severity of the cancer, the age, gender and condition of patient, and other factors well-known in the medical art. The composition may be administered once or in multiple doses.

In still another aspect, the present invention provides a method for treating cancer comprising the step of administering the composition for treating cancer to a subject suspected of having cancer. The cancer and the composition for treating cancer are the same as described above, and the composition for treating cancer coexpresses dodecameric TRAIL and suicide gene so as to synergistically increase anticancer effects. Therefore, since the therapeutic composition has anticancer therapeutic effects, it can be used in cancer therapy by administration thereof into a subject.

The composition for treating cancer administered to a subject suspected of having cancer may comprise as an active ingredient the recombinant expression vector capable of coexpressing dodecameric TRAIL and HSV-TK protein of the present invention and the recombinant adenovirus prepared by using the vector as well as the above described host cell.

As used herein, the term "subject" means a human or an animal such as horse, sheep, pig, goat, camel, antelope, dog, etc. having a disease, of which symptoms can be ameliorated by administration of the pharmaceutical composition for the treatment of cancer according to the present invention. Cancer can be effectively prevented and treated by administration of the therapeutic composition for cancer to the subject.

As used herein, the term "administration" means introduction of a predetermined substance into a subject by a certain suitable method. The composition for treating cancer according to the present invention may be administered via any of the common routes, oral or non-oral route, as long as it is able to reach a desired tissue. In addition, the therapeutic composition for cancer according to the present invention may be administered using a certain apparatus capable of transporting the active ingredients into a target cell.

The method for treating cancer of the present invention may further comprise the step of administering ganciclovir (GCV). The GCV may be administered together with the composition for treating cancer of the present invention, or before or after administration of the composition for treating cancer. In one embodiment of the present invention, the GCV was administered after administration of the stem cell therapeutic agent of the present invention, and then its effects of killing tumor cells and its therapeutic effects on cancer were evaluated.

### [Mode for Invention]

Hereinafter, the present invention will be described in more detail with reference to Examples. It will be apparent to those skilled in the art that these Examples are for illustrative purposes only, and the scope of the present invention is not intended to be limited by these Examples.

### Example 1: Construction of tPA-SPD-TRAIL DNA cassette (dTRAIL)

Codon-optimized tPA secretion signal sequence having a nucleotide sequence of SEQ ID NO. 4 and codon-optimized SPD dodecamer-forming sequence having a nucleotide sequence of SEQ ID NO. 5 were chemically synthesized in a linked form. For effective insertion into a vector, KpnI(5') and NotI(3') sites were added to its terminus. The tPA-SPD signal sequence was inserted into an inducible adenovirus shuttle vector, pShuttle-Tet10 which was digested with KpnI and NotI, so as to construct a pShuttle-Tet10/tPA-SPD vector. The inducible adenovirus shuttle vector pShuttle-Tet10 has an inducible promoter CMV-Tet10. Subsequently, codon-optimized human TRAIL (114-281) having a nucleotide sequence of SEQ ID NO. 6 was chemically synthesized. In the same manner, NotI(5') and XbaI(3') sites were added to its terminus for effective insertion into a vector. Thereafter, it was inserted into a pShuttle-Tet10/tPA-SPD vector which was digested with NotI and XbaI, so as to construct a pShuttle-Tet10/tPA-SPD-TRAIL, leading to preparation of tPA-SPD-TRAIL DNA cassette (SEQ ID NO. 1).

### Example 2: Construction of dTRAIL-IRES-TK cassette

The codon-optimized human dodecameric TRAIL sequence having a nucleotide sequence of SEQ ID NO. 1 which was prepared in Example 1, IRES sequence having a nucleotide sequence of SEQ ID NO. 2 and codon-optimized HSV-TK sequence having a nucleotide sequence of SEQ ID NO. 3 were chemically synthesized so as to construct a DNA cassette (FIG. 1). The DNA cassette thus constructed was inserted into an adenovirus shuttle vector pShuttle so as to construct a pShuttle/dTRAIL-IRES-TK vector. pShuttle/dTRAIL-IRES-TK was cleaved with PmeI, and was subjected to homologous recombination with a pAd/Easy vector incapable of self-replication in competent cells. Thereafter, an antibiotic Kanamycin was used to select pAd/dTRAIL-IRES-TK having the dTRAIL-IRES-TK cassette, which was cleaved with PacI to examine a DNA band of 35+4.5kb for accurate confirmation. pAd/dTRAIL-IRES-TK was transformed into a packaging cell 293 cell line. After 10 days, a recombinant adenovirus rAd/dTRAIL-IRES-TK expressing the dTRAIL-IRES-TK cassette was obtained.

### Example 3: Test on TRAIL and HSV-TK expressions by MSC/dTRAIL-TK

To prepare MSC/dTRAIL-TK which is a MSC expressing both dTRAIL and HSV-TK, rAd/dTRAIL-IRES-TK prepared in Example 2 and iron ions were used to transduce rat bone marrow-derived MSCs (rBM-MSC) for 30 minutes. 24 hours after transduction, Western blotting was carried out using a cell lysate to examine human TRAIL expression, and HSV-TK expression was examined by RT-PCR (FIG. 2). As a result, it was found that the prepared MSC/dTRAIL-TK expressed both TRAIL and HSV-TK.

### Example 4: Effect of ganciclovir (GCV) treatment on cell survival rate and TRAIL expression level of MSC/dTRAIL-TK

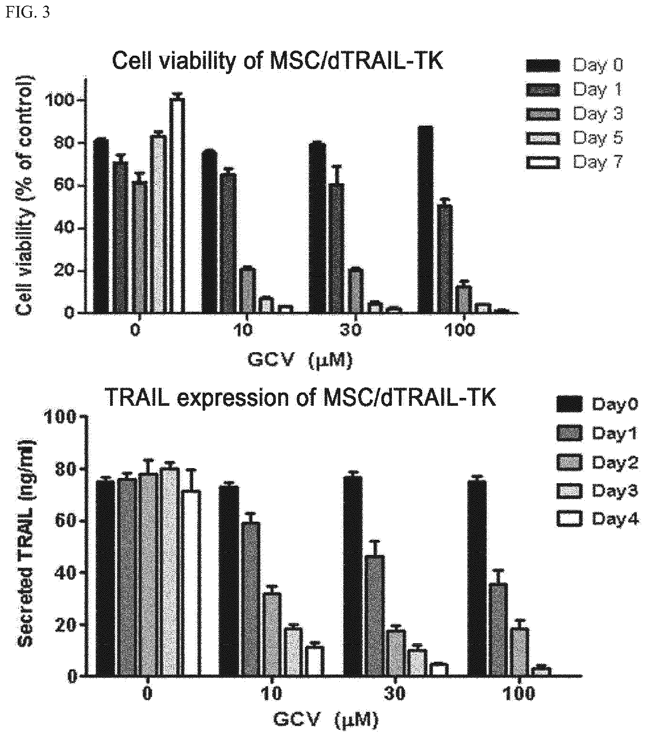

In order to examine the suicide induction effect of MSC/dTRAIL-TK by GCV treatment, cells were treated with 10, 30, and 100 µM of GCV. From 1 day after GCV treatment, survival rate of MSC/dTRAIL-TK was examined by an MTS method every other day. As a result, almost all cells were eliminated one week after treatment of 100 µM of GCV (FIG. 3).

Next, in order to examine whether TRAIL expression is reduced by MSC/dTRAIL-TK apoptosis, GCV was treated at the same concentrations, and the culture broth was collected every 24 hours to examine TRAIL expression by ELISA (enzyme-linked immunosorbent assay). As a result, it was found that TRAIL expression was reduced according to the death of engineered MSCs by GCV/TK treatment (FIG. 3).

### Example 5: Remarkably increased tumor cell death by coexpression of dTRAIL and HSV-TK in vitro

In order to examine whether MSC/dTRAIL-TK shows a combinatory (synergistic) tumoricidal effects on tumor cells in vitro, tumor cells and MSC/dTRAIL-TK were co-cultured and the apoptosis of tumor cells was analyzed. For comparison, MSC/Mock expressing none, MSC/TK expressing HSV-TK only, MSC/dTRAIL expressing dTRAIL only were co-cultured as control groups. To examine tumor-specific apoptosis, murine renal cell carcinoma RENCA cells were labeled with CFSE (Carboxyfluorescein succinimidyl ester) and co-cultured with MSCs for 48 hours, followed by 100 µM GCV treatment. The cells were then further cultured for 48 or 72 hours. Then, all cells were collected, and stained with Annexin-V and 7-AAD, and then cell death was analyzed by FACS (fluorescence activated cell sorter). As a result, tumor cell death was remarkably increased RENCA cells co-cultured with MSC/dTRAIL-TK, compared those co-cultured with MSC/dTRAIL or MSC/TK. In particular, 90% or more of tumor cells were eliminated after 72 hours of GCV treatment (FIG. 4). These results show that coexpression of dodecameric TRAIL and HSV-TK by MSC/dTRAIL-TK of the present invention result incombinatory effects on apoptosis induction of tumor cells.

### Example 6: Evaluation of therapeutic efficacy of MSC/dTRAIL-TK in metastatic cancer mouse model

In order to evaluate whether the anticancer effects of MSC/dTRAIL-TK demonstrated in vitro are also found in metastatic carcinoma mouse model, mice were injected with 5x10 of RENCA cells intravenously, and after 7 days, the same number of MSC/dTRAIL-TK was injected via the same route. For comparison, MSC/EGFP, MSC/dTRAIL, and MSC/TK were used as control groups. 2 days after MSC injection, 50 mg/kg of GCV was intraperitoneally injected for 7 consecutive days. On day 14 after tumor injection, the number of metastatic tumor nodules on the lung was counted. As a result, 35% reduction in the number of lung tumor nodules was observed in the MSC/dTRAIL-TK-treated group, displaying superior efficacy compared to MSC/TK and MSC/dTRAIL-treated groups (FIG. 5). The survival rate of the metastatic carcinoma mouse was continuously examined. As a result, mice treated with MSC/dTRAIL-TK survived up to 80 days (FIG. 6). This result suggests that MSC/dTRAIL-TK of the present invention also shows excellent anticancer effects on animal models in vivo.

### Example 7: 100% complete remission rate of metastatic cancer by repeated administration of MSC/dTRAIL-TK

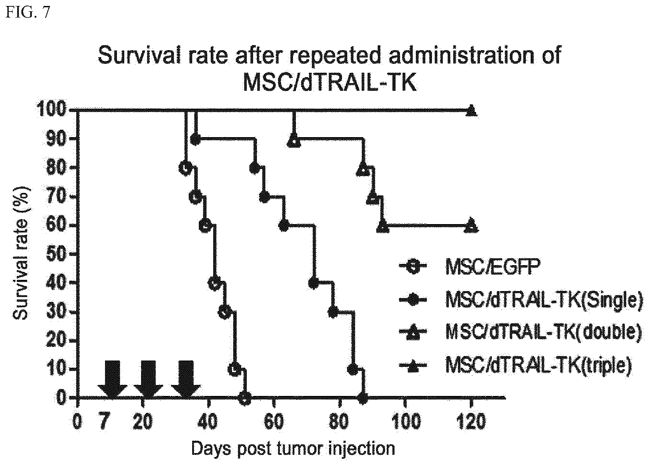

In order to examine whether anti-metastatic effects of MSC/dTRAIL-TK can be improved by repeated administrations, the metastatic carcinoma mouse model used in Example 6 was administered with MSC/dTRAIL-TK on day 7 after tumor injection at two-week intervals twice or three times. The survival rate of the metastatic carcinoma mice was examined. As a result, when administered once, all metastatic carcinoma mice died before 90 days. In contrast, when administered twice, 70% of the metastatic carcinoma mice were completely cured (FIG. 7). In addition, when mice were administered with MSC/dTRAIL-TK three times, 100% of tumor-bearing mice were completely cured. It is worth noting that complete regression of metastatic tumors is hardly achieved by conventional chemo- or radiotherapy, and more importantly, there has been no report about achieving 100% complete remission rate of the metastatic tumors using stem cell-based gene therapy alone. Therefore, it can be seen that the present invention provides very excellent and outstanding results.
## Claims

1. A stem cell coexpressing dodecameric TNF related apoptosis inducing ligand (TRAIL) and a suicide gene.

2. The stem cell according to claim 1, wherein the stem cell comprises a nucleotide sequence encoding dodecameric TRAIL and a suicide gene nucleotide sequence.

3. The stem cell according to claim 2, wherein the nucleotide sequence encoding dodecameric TRAIL comprises a nucleotide sequence encoding a secretion signal sequence, a nucleotide sequence encoding a dodecamer-forming domain, and a nucleotide sequence encoding TRAIL.

4. The stem cell according to claim 3, wherein the secretion signal sequence is tissue plasminogen activator (tPA).

5. The stem cell according to claim 3, wherein the dodecamer-forming domain is surfactant protein D (SPD).

6. The stem cell according to claim 3, wherein the TRAIL is an extracellular domain corresponding to amino acid residues at positions 114 to 281 of the full amino acid sequence of TRAIL.

7. The stem cell according to claim 1, wherein the suicide gene is selected from the group consisting of Herpes simplex virus thymidine kinase (HSV-TK), Cytosin deaminase, Nitroreductase, Carboxylesterase, Cytochrome P450 and Purine nucleoside phosphorylase (PNP).

8. The stem cell according to claim 1, wherein the suicide gene is Herpes simplex virus thymidine kinase (HSV-TK).

9. The stem cell according to claim 2, wherein the nucleotide sequence encoding dodecameric TRAIL comprises a nucleotide sequence of SEQ ID NO. 1.

10. The stem cell according to claim 8, wherein the nucleotide sequence encoding HSV-TK comprises a nucleotide sequence of SEQ ID NO. 2.

11. The stem cell according to claim 1, wherein the stem cell is a mesenchymal stem cell.

12. A composition for use in treating cancer, comprising the stem cell of any one of claims 1 to 11.

## Patentansprüche

1. Stammzelle, die dodekameren TNF-verwandten apoptoseinduzierenden Ligand (engl., TNF-related apoptosis inducing ligand, TRAIL) und ein Selbstmordgen koexprimiert.

2. Stammzelle nach Anspruch 1, wobei die Stammzelle einen nukleotidsequenzkodierenden dodekameren TRAIL und eine Selbstmordgen-Nukleotidsequenz umfasst.

3. Stammzelle nach Anspruch 2, wobei der nukleotidsequenzkodierende dodekamere TRAIL eine Nukleotidsequenz, die eine Sekretionssignalsequenz kodiert, eine Nukleotidsequenz, die eine dodekamerbildende Domäne kodiert, und eine Nukleotidsequenz, die TRAIL kodiert, umfasst.

4. Stammzelle nach Anspruch 3, wobei die Sekretionssignalsequenz Gewebeplasminogenaktivator (tPA) ist.

5. Stammzelle nach Anspruch 3, wobei die dodekamerbildende Domäne Surfactant-Protein D (SPD) ist.

6. Stammzelle nach Anspruch 3, wobei der TRAIL eine extrazelluläre Domäne ist, die den Aminosäureresten an den Positionen 114 bis 281 der vollen Aminosäuresequenz von TRAIL entspricht.

7. Stammzelle nach Anspruch 1, wobei das Selbstmordgen aus der Gruppe ausgewählt ist, die aus Herpes-simplex-Virus-Thymidinkinase (HSV-TK), Cytosindesaminase, Nitroreduktase, Carboxylesterase, Cytochrom P450 und Purinnukleosidphosphorylase (PNP) besteht.

8. Stammzelle nach Anspruch 1, wobei das Selbstmordgen Herpes-simplex-Virus-Thymidinkinase (HSV-TK) ist.

9. Stammzelle nach Anspruch 2, wobei der nukleotidsequenzkodierende dodekamere TRAIL eine Nukleotidsequenz von SEQ-ID-Nr. 1 umfasst.

10. Stammzelle nach Anspruch 8, wobei die nukleotidsequenzkodierende HSV-TK eine Nukleotidsequenz von SEQ-ID-Nr. 2 umfasst.

11. Stammzelle nach Anspruch 1, wobei die Stammzelle eine mesenchymale Stammzelle ist.

12. Zusammensetzung zur Benutzung in der Behandlung von Krebs, umfassend die Stammzelle nach einem der Ansprüche 1 bis 11.

## Revendications

1. Cellule souche co-exprimant le ligand induisant l'apoptose associé au TNF (TRAIL) dodécamère et un gène suicide.

2. Cellule souche selon la revendication 1, dans laquelle la cellule souche comprend une séquence nucléotidique codant pour TRAIL dodécamère et une séquence nucléotidique du gène suicide.

3. Cellule souche selon la revendication 2, dans laquelle la séquence nucléotidique codant pour TRAIL dodécamère comprend une séquence nucléotidique codant pour une séquence de signal de sécrétion, une séquence nucléotidique codant pour un domaine de formation de dodécamère et une séquence nucléotidique codant pour TRAIL.

4. Cellule souche selon la revendication 3, dans laquelle la séquence de signal de sécrétion est un activateur de plasminogène tissulaire (tPA).

5. Cellule souche selon la revendication 3, dans laquelle le domaine de formation de dodécamère est une protéine tensioactive D (SPD).

6. Cellule souche selon la revendication 3, dans laquelle le TRAIL est un domaine extracellulaire correspondant à des résidus d'acides aminés aux positions 114 à 281 de la séquence d'acides aminés totale de TRAIL.

7. Cellule souche selon la revendication 1, dans laquelle le gène suicide est sélectionné dans le groupe constitué par la thymidine kinase du virus de l'herpès simplex (HSV-TK), la cytosine désaminase, la nitroréductase, la carboxylestérase, le cytochrome P450 et la purine nucléoside phosphorylase (PNP).

8. Cellule souche selon la revendication 1, dans laquelle le gène suicide est la thymidine kinase du virus de l'herpès simplex (HSV-TK).

9. Cellule souche selon la revendication 2, dans laquelle la séquence nucléotidique codant pour TRAIL dodécamère comprend une séquence nucléotidique de SEQ ID NO. 1.

10. Cellule souche selon la revendication 8, dans laquelle la séquence nucléotidique codant pour HSV-TK comprend une séquence nucléotidique de SEQ ID NO. 2.

11. Cellule souche selon la revendication 1, dans laquelle la cellule souche est une cellule souche mésenchymateuse.

12. Composition pour une utilisation dans le traitement d'un cancer, comprenant la cellule souche selon l'une quelconque des revendications 1 à 11.
